# EUROPEAN PATENT SPECIFICATION

(11) **EP 1 979 039 B1**
(45) Date of publication and mention of the grant of the patent: **03.06.2015**
(21) Application number: 07763159.6
(22) Date of filing: 05.02.2007
(51) Int. Cl.: A61F 2/12, A61F 5/00

(54) **GASTRO-INTESTINAL DEVICE**
GASTROINTESTINALE VORRICHTUNG
DISPOSITIF GASTRO-INTESTINAL

(30) Priority: 03.02.2006 US 764673 P
(43) Date of publication of application: 15.10.2008
(73) Proprietor: BARONOVA, INC., Goleta, CA 93117 (US)
(72) Inventor: BURNETT, Daniel R., San Francisco, CA 94131 (US)
(74) Representative: Miller, James Lionel Woolverton
(86) International application number: PCT/US2007/003260
(87) International publication number: WO 2007/092501

(56) References cited:
- WO-A1-90/00369
- US-A- 4 878 905
- US-A1- 2003 040 804
- US-A1- 2005 055 039
- US-A1- 2005 055 039
- US-B2- 6 675 809

## Description

### BACKGROUND OF THE INVENTION

### 1. Field of the Invention

The present invention concerns a gastro-intestinal device for treating addiction and other medical conditions. More particularly, the present invention concerns a device that is positioned orally, nasally, or transcutaneously in a patient's gastro-intestinal tract and that, in some embodiments, delivers medications and/or noxious stimuli over extended periods of time.

### 2. Description of the Related Art

Alcoholism is an addictive condition that results in severe debilitation and that destroys the lives of millions of people every year.

Therapies known in the prior art for treating alcoholism involve counseling and enrollment in support groups, but counseling-based therapies have proven to be largely ineffective, as shown by the rate of recidivism for patients that have undergone such therapies.

Pharmacological therapies have also been attempted, which involve the prescription of one or more medications that the patient must ingest form time to time. One pharmacological therapy involves the prescription of antieuphorics, such as naltrexone, which reduce the euphoria induced by alcohol and which also reduce cravings during the withdrawal phase. Another pharmacological therapy involves the prescription of antitolerance agents, such as antabuse agents or tetraethylthiuram disulfide (known in the medical trade as disulfuram), which cause a range of unpleasant symptoms upon the ingestion of alcohol. These pharmacological agents may be effective if taken as prescribed but turn out to be ineffective in the majority of cases because their intake is typically not controlled and patients are free to forego their ingestion and return to their destructive behavior. Moreover, each of these medications may cause undesirable collateral effects.

In addition to alcoholism, dependency from many other harmful substances has been treated with therapies that have proven to be either ineffective or of limited duration. In addition to substance abuse, food abuse and obesity has been identified as major health problems that lead to a variety of illnesses and that decrease self-esteem, but treatments proposed to date typically provide only temporary benefits, because the patient can decide when to discontinue the treatment, or is unable to self-discipline herself to undergo the rigorous regimen required to overcome her condition. In order to reduce weight, patients sometimes resort to surgical procedures that are not only risky and expensive, but that sometimes require a long recuperative process.

US2003/0040804 A1 discloses a device for inducing weight loss in a patient which includes a tubular prosthesis self-expandable from a collapsed position to an expanded position.

Therefore, there is a need for a therapy that effectively treats substance addiction and abuse and that is not prone to altering or tampering by the patient.

Further, there is a need for a therapy that effectively treats substance addiction and abuse, and that either reduces the euphoria associated with substance abuse or that causes an adverse reaction discouraging the addictive behavior.

Still further, there is a need for a therapy that effectively treats substance addiction and abuse and that may be reversible, transient in nature, and implemented with non-invasive or minimally invasive procedures.

### BRIEF SUMMARY OF THE INVENTION

According to one aspect of the present invention, there is provided a device for treating obesity and/or addiction according to Claim 1.

The present invention concerns a device that can be positioned in a patient's gastrointestinal tract and that, in certain embodiments, delivers oral medications and/or noxious stimuli over an extended period of time. The device of the present invention can be inserted and removed orally, nasally or transcutaneously and, in certain embodiments, expands reversibly once in the gastro-intestinal space.

In one embodiment, the device of the present invention is configured for treating alcoholism by delivering an anti-alcoholic agent at a predetermined rate and in a format that prevents patient tampering while providing the required daily amount of the agent. This embodiment may deliver any medicine absorbable across the gastro-intestinal tract and may include a retaining structure that is lockable and/or made of shape-memory materials, so to maintain the device in the desired configuration and condition, and confined within the gastro-intestinal space.

In this embodiment of the invention, the device is inserted orogastrically and expanded in the gastric space. The retaining structure is made of or coated with a biocompatible material that resists the harsh gastric environment; for example, the retaining structure may be manufactured from a silicone coated shape memory wire, or from a biocompatible metals rubber, polymer, etc. Further, the retaining structure may have a spherical shape, or any other shape that can be manufactured other shape produced from a polymer infused into a collapsible, preformed casing inside the gastric space. Such a casing will degrade over time exposing the polymer or may be extracted from the gastro-intestinal tract. Because the polymer is infused with a drug or other curative substance, the polymer structure will elute the drug or the curative substance over time into the intestine of the patient.

In certain embodiments of the invention, a dispensing member is positioned inside the device and includes a pump or other device suitable for delivering or eluting a drug into the gastro-intestinal tract. In another embodiments, the device includes one or more sensors coupled to a generator of noxious stimuli, so that, once the sensors detects one or more predetermined addictive substances, a noxious stimulus is generated, causing a negative feedback signal that becomes associated with the addictive substance. The noxious stimulus may be a vibratory sensation or an audible noise, or may trigger the release of a quick-acting, nausea-generating substance or the generation of uncomfortable electrical impulses into the stomach or the intestine. In still other embodiments, the device may be positioned transpylorically and includes a central portion spanning across the duodenal orifice and connecting two end portions disposed on opposing sides of the duodenal orifice.

In yet other embodiments, the retaining structure may include no dispensing member and may be employed for treating obesity by providing a sense of fullness in the stomach and by reducing appetite accordingly.

Methods of use of the present device for the treatment of alcoholism and other medical conditions are also described herein.

### BRIEF DESCRIPTION OF THE SEVERAL VIEWS OF THE DRAWINGS

The drawings constitute a part of this specification and illustrate exemplary embodiments of the invention, which may be embodied in various forms. It is to be understood that in some instances various aspects of the invention may be shown exaggerated or enlarged to facilitate an understanding of the invention.
FIGS. 1A-1C illustrate side views of a first embodiment of the invention that includes a foldable reversible member.
FIGS. 2A-2B illustrate top views of the embodiment of FIGS. 1A-1C, showing the reversible member in the elongated and contracted configurations.
FIGS. 3A-3C illustrate top views of the embodiment of FIGS. 1A-1C, in which the reversible member transitions form the contracted configuration to the elongated configuration.
FIGS. 4A-4B illustrate top views of the embodiment of FIGS. 1A-1C, showing the reversible member coupled with the dispensing member.

### DETAILED DESCRIPTION OF EMBODIMENTS OF THE INVENTION

Detailed descriptions of embodiments of the invention are provided herein. It is to be understood, however, that the present invention may be embodied in various forms. Therefore, the specific details disclosed herein are not to be interpreted as limiting, but rather as a representative basis for teaching one skilled in the art how to employ the present invention in virtually any detailed system, structure, or manner.

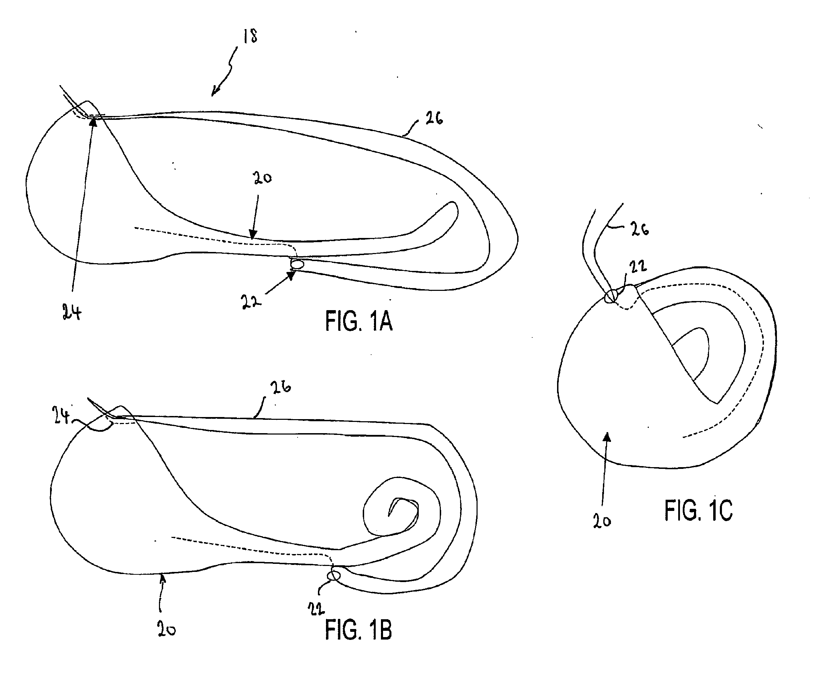

Referring first to FIGS. 1A-1C, there is shown a first embodiment of the invention. Intragastric device 18 includes reversible member 20 that can be positioned in a patient's stomach or in other parts of the gastro-intestinal tract and that can assume an extended configuration before introduction in the stomach, as shown in FIG. 1A. Reversible member 20 can then be refolded after positioning in the stomach, as shown in FIGS. 1B and 1C.

Locking button 22 is connected to a tether and is used to force reversible member 20 to fold on itself and to maintain the folded configuration. More particularly, locking button 22 is progressively pulled through locking lumen 24 by pulling on both ends of string 26, causing locking button 22 to traverse locking lumen 24 while reversible member 20 progressively assumes a contracted, essentially spherical configuration. After locking button 22 has completely traversed locking lumen 24 and reversible member 20 is securely formed in the contracted configuration, string 26 can be removed by pulling on one end of it and by having string 26 rotate around and release from locking button 22. Alternatively, string 26 may be removed by cutting it in the proximity of locking button 22 with the use of endoscopic tools, or with the use of other medical or surgical techniques known in the art.

Intragastric device 18 may be inserted in a patient's stomach or in other parts of the gastro-intestinal tract to provide a variety of gastric-related benefits, including the reduction and eradication of an addiction by providing, for example, gastric stimulation, drug release, or noxious stimuli. Additionally, intragastric device 18 may be used with or without a pylorus-spanning element and a duodenal bulb extending from reversible member 20, as described in greater detail below. The specific embodiment of intragastric device 18 illustrated in FIGS. 1A-1C does not include a pylorus-spanning element.

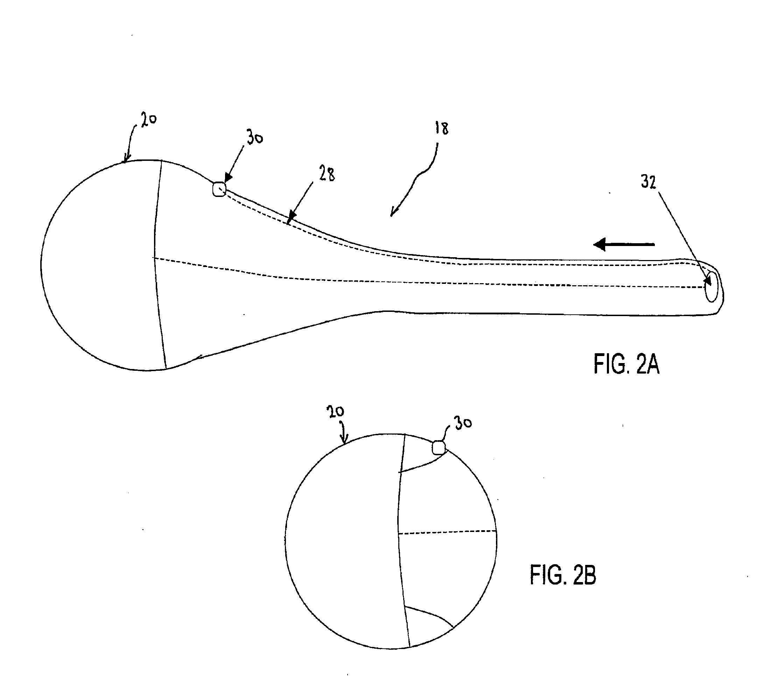

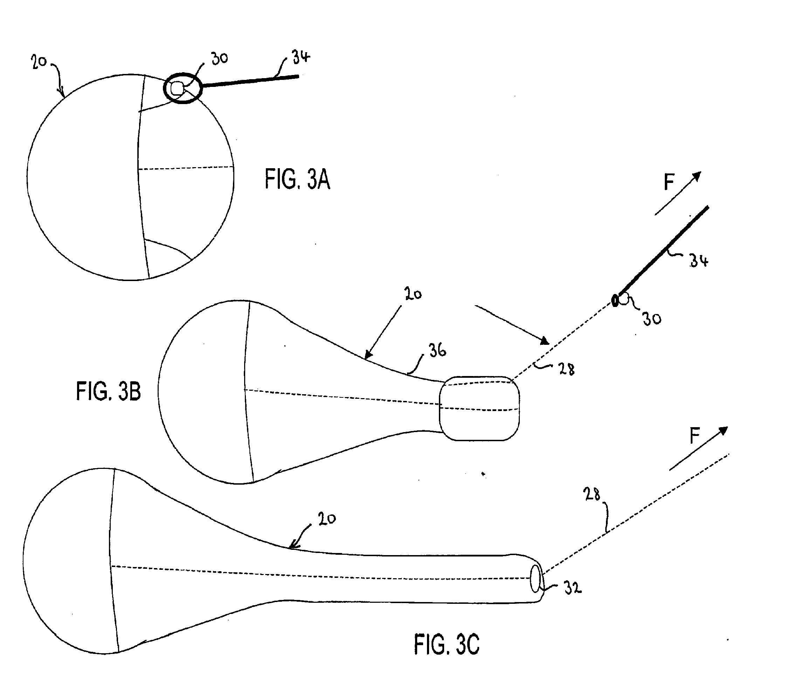

Referring now to FIGS 2A-2B and 3A-3C, one example of the removal features of intragastric device 18 is described. Retrieval tether 28 is connected to retrieval ball 30 and is cast loosely in the periphery of reversible member 20, and more firmly in the central portion of reversible member 20. Retrieval ball 30 may be the same as locking button 22 or may be a separate component of intragastric device 20. Anchor 32 may also be provided, to prevent an undesired tearing of intragastric device 20 when tension is applied to retrieval ball 30.

In the illustrated example, intragastric device 18 may be removed by snaring retrieval ball 30 (attached to retrieval tether 28) with snare 34, causing retrieval tether 28 to open rip-strip 36 and further causing reversible member 20 to assume an unfolded configuration suited for removal through the esophagus. Conversely, this and other embodiments of intragastric device 18 may include a locking feature that will prevent the unfolding of reversible member 20 in the gastric space and that will require endoscopic manipulation for removal, in order to unlock reversible member 20 and for extracting it through the esophagus or through surgery.

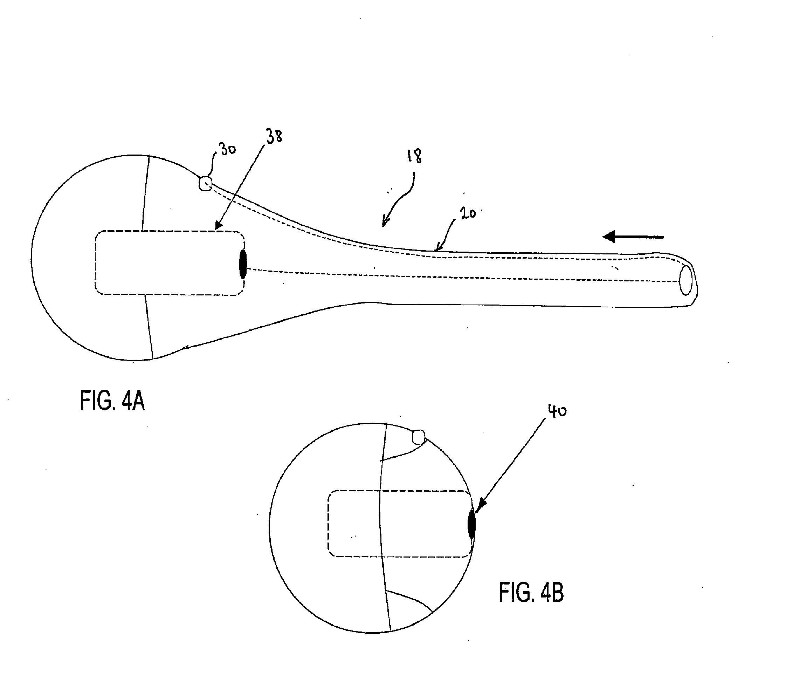

Referring now to FIGS 4A-4B, intragastric device 18 also includes dispensing member 38, which is disposed within reversible member 20 and which can provide a drug or a stimulus (for example, a vibratory, acoustic stimulus, or electric stimulus) to the patient's stomach or gastro-intestinal tract. One skilled in the art will appreciate that other types of stimuli may also be provided, which are all included within the scope of the present invention.

A plurality of ancillary components, such as sensors and actuators, also may be included in intragastric device 18. For example, sensors may be included that are capable of sensing virtually any substance, including substances of addiction. Actuators also may be included, such as drug pumps or other release devices that provide a constant or sensor-triggered elution of a drug and/or noxious stimulus, for example, a vibratory, auditory, or electrical shock signal, or the release a short-acting noxious compound. Intragastric device 18 may also incorporate refilling/recharging port 40, designed to engage endoscopic tools and/or a specially designed catheter. Such a catheter may be magnetically tipped and may engage intragastric device 18 in a variety of ways. In addition, intragastric device 18 may be recharged, programmed or interrogated either through the catheter mechanism or with an external signal/power generator and/or receiver, connected transcutaneously or wirelessly to intragastric device 18. Communication devices, data receivers, data storage modules, microprocessors and rechargeable power sources also may be included in intragastric device 18.

Any suitable materials may be used to produce the embodiment of the invention described herein. In one type of embodiment, for example, a gastric device may comprise an expandable balloon or casing fabricated from silicone, silicone elastomers, latex, or hydrogels that typically expand upon contact with fluids, may be utilized as polymers within the device.

In all gastric embodiments, the proximal portion of the gastric device has a supportive or structural function and a large enough cross sectional diameter that prevents passage of the device through the pyloric valve. The distal portion of the gastric device typically contacts the pyloric valve and/or tissue adjacent the pyloric valve, intermittently and/or partially blocking the valve or providing electrical stimulation.

In some embodiments, the distal portion of the gastric device is made of a compliant material, so that it does not harm the tissue when it contacts stomach tissue in, around or adjacent the pyloric valve. Also in some embodiments, the proximal and distal portions are produced from the same material, with the proximal portion having a greater amount of material, greater wall thickness or similar structural features relative to the distal portion. Alternatively, any of the above-described embodiments could be altered to allow for anchoring in the duodenal bulb. As with the pyloric sphincter in the stomach, the duodenal bulb necks down to a smaller lumen, creating an effective sphincter.

Any of the previously described embodiments can be configured to provide for retention in the duodenal bulb without an excessively firm stenting or without puncturing the intestinal wall. In fact, any of the previously described embodiments can be endoscopically delivered and removed, and can be retained within the stomach and/or the duodenum without attachment to the wall of the gastro-intestinal tract. Therefore, the present invention enables an effective stimulation of the intestine without the need for a gastric component.

Likewise, an embodiment of the present invention could be used to anchor a gastrointestinal energy delivery device in any region of the gastro-intestinal tract where there is a decrease in the diameter of the lumen that is sufficient to maintain an interference fit. This includes the pharynx, the esophagus (upper, cardiac and lower sphincters), the pylorus, the duodenal bulb, the ileocecal valve, the rectum and any other region with a change in diameter sufficient for anchoring a stimulating device through an interference fit.

As previously discussed, a gastric device according to the present invention may be covered by an erodable or biodegradable covering for delivery into the stomach. Such a covering may be configured to constrain the device, and may naturally break down and dissolve after the covering comes into contact with substances in the gastric lumen, thus releasing the device and allowing it to expand. In one embodiment, a gastric device according to the present invention may be covered by different materials each configured to erode at differing rates or in different chemical environments within the stomach.

Also as previously discussed, any of the embodiments described hereinbefore and illustrated in FIGS. 1-4B may include one or more dispensing members that generate noxious stimuli, one or more sensors, or a combination of both. Such dispensing members and sensors may be coupled with any portion of an anchoring device, pyloric corking device or the like, for example, any portions of the gastric device that reside in the stomach, span the pyloric valve or are disposed within the duodenum. In some embodiments, one or more dispensing members or sensors are coupled with an anchoring device or corking device via one or more tethers, while in other embodiments all the dispensing members and/or sensors may be coupled directly to the anchoring member.

Among the actuators that may be coupled with an anchoring device is an energy transmitter that applies energy to gastrointestinal tissue, for example, one or more of radio- frequency, ultrasound, microwave, cryogenic, laser, electrical, mechanical and thermal energy. One or more substances, such as lipids, drugs, enzymes, diagnostic agents, vitamins, minerals, and the like, also may be releasably coupled with the outer surface of the device or may be housed within one or more refillable reservoirs. These substances may be detachably coupled to the anchoring device or may be disposed within the reversible member.

Still another type of actuator is a member for occupying space in the patient's stomach, so to enhance the patient's feeling of satiety. Yet another type of actuator is a trigger adapted to elicit a biological response, such as a surface coating adapted to induce a satiety response. One more type of actuator is an imaging device, but, more generally, any component suitable for performing a function within the gastrointestinal system may be coupled to an anchoring device and to a pyloric corking device, or may be disposed within the reversible member.

In some embodiments, at least one sensor may be coupled to the anchoring member for sensing one or more characteristics of the gastrointestinal tract, for example, pH, temperature, bile content, nutrient content, fats, sugars, alcohol, opiates, drugs, analytes, electrolytes, or hemoglobin. Further, a processor may be included that is adapted to process data related to the sensed signals and to provide the processed data and other signals to the at least one energy delivery member. Still further, a receiver may be included that receives data transmitted from a remote source, or a transmitter for transmitting data, or a data storage module, or a rechargeable power source, or any suitable combination thereof.

As previously discussed, in some embodiments an anchoring device and/or a generator of noxious stimulus may be delivered by means of an elongate catheter device, such as an orogastric or nasogastric tube passed through the patient's esophagus and into the stomach. The catheter device or a separate device may be employed for modifying, adjusting or recharging an anchoring or dispensing device that has been positioned in the gastro-intestinal tract, thereby enabling a modification of the gastric device without removing or replacing the device.

Further, a gastric device according to the present invention may be used to detect ingestion of an addictive substance and records exposure to an undesired substance without delivering any noxious stimuli. This embodiment may be useful in screening patients for liver or other transplants where maintaining a non-addictive behavior is critical.

The present invention is particularly applicable to the treatment of a variety of addictive conditions, such as alcoholism, and to the treatment of obesity. By using a device constructed according to the principles of the present invention, a patient is left without discretion about undergoing the prescribed regimen, because the patient is unable to manipulate the device and to prevent it from operating as planned. At the same time, a clinician can adjust the device as necessary by using, for example, one the previously described adjustment methods. A device constructed according to the principles of the present invention also can be configured to be temporary, either by incorporating removal features, as previously described, or by being manufactured from one or more materials that dissolve over time within the body system.

While the invention has been described in connection with the above described embodiments, it is not intended to limit the scope of the invention to the particular forms set forth, but on the contrary, it is intended to cover such alternative and modifications as may be included within the scope of the invention. For example, one skilled in the art will recognize that the devices described herein may be used to treat a variety of other conditions or perform a variety of other function within the gastrointestinal tract and without departing from the scope of the present invention. Therefore, the foregoing description is provided primarily for exemplary purposes and should not be interpreted to limit the scope of the invention as it is defined in the following claims.

## Claims

1. A device (18) for treating obesity and/or addiction, the device comprising:
a reversible member (20) to be disposed in a patient's gastro-intestinal tract;
a dispensing member (38) coupled to the reversible member,
wherein the reversible member is structured to have an elongated configuration during insertion into the patient's gastro-intestinal tract and a contracted configuration after the insertion; **characterised in that** the apparatus further comprises:
a locking button (22) positioned along the reversible member;
a length of string (26) slidably attached to the locking button;
wherein a distal end of the elongated configuration is coupled to a proximal end of the elongated configuration via the length of string attached to the locking button where the length of string is passed entirely through a locking lumen (24) defined through the distal end of the elongated configuration of the reversible member such that the proximal end of the elongated configuration of the reversible member is approximated into and through the distal end of the elongated configuration of the reversible member until the locking button has traversed the locking lumen into the contracted configuration of the reversible member, and
wherein the length of string is removable from the locking button by tensioning one end of the string once the reversible member is in the contracted configuration.

2. The device (18) of claim 1, wherein the reversible member (20) is to be positioned in the stomach or in the duodenal bulb.

3. The device (18) of claim 1,
wherein the distal end is caused to roll and couple with the proximal end after the insertion to form the contracted configuration,
wherein the contracted configuration is essentially spherical, and
wherein the distal end is releasable from the proximal end, providing for the reversible member (20) to revert to the elongated configuration prior to removal from the patient's gastro-intestinal tract.

4. The device of claim 1, wherein the dispensing member (38) releases one or more of a drug and a stimulus into the patient's gastro-intestinal tract after the insertion.

5. The device (18) of claim 4,
wherein the distal end has an opening,
wherein the distal end is caused to roll and couple with the proximal end after the insertion to form the contracted configuration,
wherein the contracted configuration is essentially spherical,
wherein the distal end is releasable from the proximal end, providing for the reversible member (20) to revert to the elongated configuration prior to removal from the patient's gastro-intestinal tract, and
wherein the dispensing member (38) is disposed adjacent to the proximal end in the elongated configuration, and wherein the dispensing member is connected to the opening in the contracted configuration.

6. The device (18) of claim 4, wherein the dispensing member (38) comprises a drug pump.

7. The device (18) of claim 6, wherein the drug pump provides a constant or sensor- triggered elution of a drug.

8. The device (18) of claim 4, wherein the device (18) comprises a generator of a noxious stimulus.

9. The device (18) of claim 8, wherein the noxious stimulus is a vibratory stimulus, an auditory stimulus, an electrical stimulus, or a short-acting noxious compound released into the patient's gastro-intestinal tract.

10. The device (18) of claim 4, further comprising one or more of a sensor, an actuator, a communication device, a data storage module, a microprocessor, and a rechargeable power source.

11. The device (18) of claim 4, wherein the dispensing member (38) comprises one or more of a refilling port, a recharging port, and a programming port designed to engage a catheter or an endoscopic tool.

12. The device (18) of claim 4, wherein the dispensing member (38) is coupled to an electrode disposed in the gastro-intestinal tract.

13. The device (18) of claim 4, wherein the reversible member (20) is positioned in the stomach, and wherein the device further comprises a bulb positioned in the intestinal tract and connected to the reversible member by a pylorus spanning member (252).

14. The device (18) of claim 13, wherein the dispensing member (38) releases one or more of the drug and the stimulus by releasing the one or more of the drug and the stimulus through the pylorus spanning member (252).

15. The device (18) of claim 13, wherein the pylorus spanning member (252) contracts upon contact with a predetermined substance, thereby causing the bulb to block exit from the stomach into the intestinal tract.

16. The device (18) of claim 1, wherein the reversible member (20) is made of a compliant material.

## Patentansprüche

1. Vorrichtung (18) zur Behandlung von Fettleibigkeit und/oder Abhängigkeit, wobei die Vorrichtung umfasst:
eine in dem Magen-Darm-Trakt eines Patienten anzuordnende reversierbare Einrichtung (20),
eine Abgabeeinrichtung (38), die mit der reversierbaren Einrichtung gekoppelt ist,
wobei die reversierbare Einrichtung strukturiert ist, während der Einbringung in den Magen-Darm-Trakt des Patienten eine langgestreckte Konfiguration und eine zusammengezogene Konfiguration nach der Einbringung aufzuweisen, **dadurch gekennzeichnet, dass** die Vorrichtung ferner enthält:
einen Verriegelungsknopf (22), der entlang der reversierbaren Einrichtung positioniert ist;
ein Schnurstück (26), das verschiebbar an dem Verriegelungsknopf angebracht ist;
wobei ein distales Ende der langgestreckten Konfiguration mit einem proximalen Ende der langgestreckten Konfiguration über das Schnurstück, das an dem Verriegelungsknopf angebracht ist, verbunden ist, wobei das Schnurstück vollständig durch ein Verriegelungslumen (24), das durch das distale Ende der langgestreckten Konfiguration der reversierbaren Einrichtung definiert ist, derart hindurchgeht, dass das proximale Ende der langgestreckten Konfiguration der reversierbaren Einrichtung dem distalen angenähert ist und durch es durchgeht, bis der Verriegelungsknopf das Verriegelungsvolumen in die zusammengezogene Konfiguration der reversierbaren Einrichtung durchquert hat, und
wobei das Schnurstück aus dem Verriegelungsknopf durch Ziehen an einem Ende der Schnur entfernbar ist, sobald die reversierbare Einrichtung in der zusammengezogenen Konfiguration ist.

2. Vorrichtung (18) nach Anspruch 1, wobei die reversierbare Einrichtung (20) in dem Magen oder in dem Bulbus duodeni zu positionieren ist.

3. Vorrichtung (18) nach Anspruch 1,
wobei das distale Ende nach der Einbringung zum Rollen und zum Verbinden mit dem proximalen Ende gebracht wird, um die zusammengezogene Konfiguration zu bilden,
wobei die zusammengezogene Konfiguration im Wesentlichen kugelförmig ist, und
wobei das distale Ende von dem proximalen Ende lösbar ist, was es ermöglicht, dass die reversierbare Einrichtung (20) vor der Entfernung aus dem Magen-Darm-Trakt des Patienten in die langgestreckte Konfiguration zurückkehrt.

4. Vorrichtung nach Anspruch 1, wobei die Abgabeeinrichtung (38) eines oder mehrere Arzneimittel und einen Reiz in den Magen-Darm-Trakt des Patienten nach der Einbringung freisetzt.

5. Vorrichtung (18) nach Anspruch 4,
wobei das distale Ende eine Öffnung aufweist;
wobei das distale Ende nach der Einbringung zum Rollen und zum Verbinden mit dem proximalen Ende gebracht wird, um die zusammengezogene Konfiguration zu bilden,
wobei die zusammengezogene Konfiguration im Wesentlichen kugelförmig ist,
wobei das distale Ende von dem proximalen Ende lösbar ist, was es ermöglicht, dass die reversierbare Einrichtung (20) vor der Entfernung aus dem Magen-Darm-Trakt des Patienten in die langgestreckte Konfiguration zurückkehrt, und
wobei die Abgabeeinrichtung (38) benachbart zu dem proximalen Ende in der langgestreckten Konfiguration angeordnet ist und wobei die Abgabeeinrichtung mit der Öffnung in der zusammengezogenen Konfiguration verbunden ist.

6. Vorrichtung (18) nach Anspruch 4, wobei die Abgabeeinrichtung (38) eine Arzneimittelpumpe enthält.

7. Vorrichtung (18) nach Anspruch 6, wobei die Arzneimittelpumpe eine konstante oder sensorgesteuerte Elution eines Arzneimittels bereitstellt.

8. Vorrichtung (18) nach Anspruch 4, wobei die Vorrichtung (18) einen Generator eines Übelkeits-Reizes enthält.

9. Vorrichtung (18) nach Anspruch 8, wobei der Übelkeits-Reiz ein Vibrationsreiz, ein akustischer Reiz, ein elektrischer Reiz oder eine kurzwirkende Übelkeits-Verbindung ist, die in den Magen-Darm-Trakt des Patienten freigesetzt wird.

10. Vorrichtung (18) nach Anspruch 4, ferner umfassend eines oder mehrere von einem Sensor, einem Antriebselement, einem Kommunikationsgerät, einem Datenspeichermodul, einem Mikroprozessor und einer wiederaufladbaren Stromquelle.

11. Vorrichtung (18) nach Anspruch 4, wobei die Abgabeeinrichtung (38) eines oder mehrere von einem Nachfüllanschluss, einem Aufladeanschluss und einem Programmieranschluss enthält, der für das Eingreifen eines Katheters oder eines endoskopischen Instruments gestaltet ist.

12. Vorrichtung (18) nach Anspruch 4, wobei die Abgabeeinrichtung (38) mit einer Elektrode verbunden ist, die in dem Magen-Darm-Trakt angeordnet ist.

13. Vorrichtung (18) nach Anspruch 4, wobei die reversierbare Einrichtung (20) in dem Magen positioniert ist und wobei die Vorrichtung ferner einen Ballon enthält, der in dem Darmtrakt positioniert und mit der reversierbaren Einrichtung durch ein Pylorus-durchspannendes Element (252) verbunden ist.

14. Vorrichtung (18) nach Anspruch 13, wobei die Abgabeeinrichtung (38) ein oder mehrere Arzneimittel und den Reiz freisetzt durch die Freisetzung des einen oder der mehreren Arzneimittel und des Reizes durch das Pylorus-durchspannende Element (252).

15. Vorrichtung (18) nach Anspruch 13, wobei das Pylorus-durchspannende Element (252) sich beim Kontakt mit einer vorbestimmten Substanz zusammenzieht, wodurch der Ballon veranlasst wird, den Ausgang von dem Magen in den Darmtrakt zu schließen.

16. Vorrichtung (18) nach Anspruch 1, wobei die reversierbare Einrichtung (20) aus einem nachgiebigen Material hergestellt ist.

## Revendications

1. Dispositif (18) destiné à traiter l'obésité et/ou une addiction, le dispositif comprenant :
un élément réversible (20) qui doit être disposé dans le tractus gastro-intestinal d'un patient ;
un élément de distribution (38) couplé à l'élément réversible ;
dans lequel l'élément réversible est structuré pour avoir une configuration allongée durant l'insertion dans le tractus gastro-intestinal du patient et une configuration contractée après l'insertion ; **caractérisé en ce que** l'appareil comprend en outre :
un bouton de verrouillage (22) positionné le long de l'élément réversible ;
une longueur de cordon (26) attachée de manière coulissante au bouton de verrouillage ;
dans lequel une extrémité distale de la configuration allongée est couplée à une extrémité proximale de la configuration allongée via la longueur du cordon attaché au bouton de verrouillage, où la longueur du cordon passe entièrement à travers une lumière de verrouillage (24) définie à travers l'extrémité distale de la configuration allongée de l'élément réversible de telle sorte que l'extrémité proximale de la configuration allongée de l'élément réversible se rapproche dans et à travers l'extrémité distale de la configuration allongée de l'élément réversible jusqu' à ce que le bouton de verrouillage traverse la lumière de verrouillage dans la configuration contractée de l'élément réversible, et
dans lequel la longueur du cordon est détachable du bouton de verrouillage sous l'action d'une tension sur une extrémité du cordon une fois que l'élément réversible est dans la configuration contractée.

2. Dispositif (18) selon la revendication 1, dans lequel l'élément réversible (20) doit être positionné dans l'estomac ou dans le bulbe duodénal.

3. Dispositif (18) selon la revendication 1,
dans lequel l'extrémité distale est amenée à s'enrouler et s'accoupler avec l'extrémité proximale après l'insertion pour former la configuration contractée,
dans lequel la configuration contractée est essentiellement sphérique, et
dans lequel l'extrémité distale est libérable de l'extrémité proximale, ce qui permet à l'élément réversible (20) de revenir dans la configuration allongée avant d'être enlevé du tractus gastro-intestinal du patient.

4. Dispositif selon la revendication 1, dans lequel l'élément de distribution (38) libère un élément ou plus parmi un médicament et un stimulus dans le tractus gastro-intestinal du patient après l'insertion.

5. Dispositif (1 8) selon la revendication 4,
dans lequel l'extrémité distale a un orifice,
dans lequel l'extrémité distale est amenée à s'enrouler et s'accoupler avec l'extrémité proximale après l'insertion, pour former la configuration contractée,
dans lequel la configuration contractée est essentiellement sphérique,
dans lequel l'extrémité distale est libérable de l'extrémité proximale, ce qui permet à l'élément réversible (20) de revenir dans la configuration allongée avant d'être enlevé du tractus gastro-intestinal du patient, et
dans lequel l'élément de distribution (38) est disposé de manière adjacente à l'extrémité proximale dans la configuration allongée, et dans lequel l'élément de distribution est connecté à l'orifice dans la configuration contractée.

6. Dispositif (18) selon la revendication 4, dans lequel l'élément de distribution (38) comprend une pompe à médicaments.

7. Dispositif (18) selon la revendication 6, dans lequel la pompe à médicaments fournit une élution d'un médicament de manière constante ou déclenchée par capteur.

8. Dispositif (18) selon la revendication 4, dans lequel le dispositif (18) comprend un générateur de stimulus nocif.

9. Dispositif (18) selon la revendication 8, dans lequel le stimulus nocif est un stimulus vibratoire, un stimulus auditif, un stimulus électrique ou un composé nocif à action rapide libéré dans le tractus gastro-intestinal du patient.

10. Dispositif (18) selon la revendication 4, comprenant en outre un élément ou plus parmi un capteur, un actionneur, un système de communication, un module de stockage de données, un microprocesseur et une source d'énergie rechargeable.

11. Dispositif (18) selon la revendication 4, dans lequel l'élément de distribution (38) comprend un élément ou plus parmi un port de re-remplissage, un port de rechargement et un port de programmation conçu pour engager un cathéter ou un outil endoscopique.

12. Dispositif (18) selon la revendication 4, dans lequel l'élément de distribution (38) est couplé à une électrode disposée dans le tractus gastro-intestinal.

13. Dispositif (18) selon la revendication 4, dans lequel l'élément réversible (20) est positionné dans l'estomac, et dans lequel le dispositif comprend en outre un bulbe positionné dans le tractus intestinal et connecté à l'élément réversible par un élément de portée de pylore (252).

14. Dispositif (18) selon la revendication 13, dans lequel l'élément de distribution (38) libère un élément ou plus parmi le médicament et le stimulus en libérant l'un ou plusieurs des éléments parmi le médicament et le stimulus par l'intermédiaire de l'élément de portée de pylore (252).

15. Dispositif (18) selon la revendication 13, dans lequel l'élément de portée de pylore (252) se contracte lors du contact avec une substance prédéterminée, amenant ainsi le bulbe à bloquer la sortie de l'estomac dans le tractus intestinal.

16. Dispositif (18) selon la revendication 1, dans lequel l'élément réversible (20) est composé d'un matériau conforme.
